# EUROPEAN PATENT SPECIFICATION

(11) **EP 2 399 513 B1**
(45) Date of publication and mention of the grant of the patent: **04.01.2017**
(21) Application number: 10006537.4
(22) Date of filing: 23.06.2010
(51) Int. Cl.: A61B 5/11, A61B 5/00, G06F 19/00, G06K 9/00

(54) **System for non-invasive automated monitoring, detection, analysis, characterisation, prediction or prevention of seizures and movement disorder symptoms**
System zur automatischen nichtinvasiven Überwachung, Detektion, Analyse, Kennzeichnung, Vorhersage oder Vorbeugung von Anfällen und Bewegungsstörungssymptomen
Système pour la surveillance automatique non invasive, la détection, analyse, caractérisation, prédiction ou prévention des crises et symptômes des troubles du mouvement

(43) Date of publication of application: 28.12.2011
(73) Proprietor: QATAR UNIVERSITY QSTP-B, Doha (QA); Cornell University, Ithaca, NY 14850 (US)
(72) Inventor: Khattab, Tamer, Doha (QA); Mohamed, Amr, Doha (QA); Abu-Dayya, Adnan, Doha (QA); Uthman, Basim, Doha (QA); Streletz, Leopold, Doha (QA)
(74) Representative: JENSEN & SON

(56) References cited:
- EP-A2- 2 123 221
- WO-A1-02/082999
- WO-A1-2009/020880
- WO-A2-2007/034476

## Description

The present invention relates to a monitoring system for predicting, detecting, analysing, characterising, preventing or subduing movement disorders or transient neurological disorders, in particular seizures associated with syndromes and diseases, such as epilepsy, Parkinson's disease, severe asthma attacks or other conditions leading to hypoxia, different types of dystonia or dyskinesia, Wilson's disease, Pitt-Hopkins syndrome and others. Generally, the term "seizure" is used almost exclusively to refer to an epileptic seizure; however, its more general meaning will be used herein, i.e. it will be used to refer to any type of seizure.

A movement disorder is a neurological condition that affects the speed, fluency, quality, and ease of movement. Abnormal fluency or speed of movement (dyskinesia) may involve excessive or involuntary movement (hyperkinesia), slowed or absent voluntary movement (hypokinesia). Movement disorders include the following conditions: ataxia (lack of coordination, often producing jerky movements), dystonia (causes involuntary movement and prolonged muscle contraction), Huntington's disease (also called chronic progressive chorea), multiple system atrophies (e.g., Shy-Drager syndrome), myoclonus (rapid, brief, irregular movement), Parkinson's disease, progressive supranuclear palsy (rare disorder that affects purposeful movement), restless legs syndrome (RLS), reflex sympathetic dystrophy/periodic limb movement disorder (RSD/PLMD), tics (involuntary muscle contractions), Tourette's syndrome, tremor (e.g., essential tremor, resting tremor) and Wilson disease (inherited disorder that causes neurological and psychiatric symptoms and liver disease). On the other hand, a seizure is a sudden episode of transient neurologic symptoms caused by abnormal electrical activity in the brain. Typically, symptoms can include involuntary muscle movements, sensory disturbances or altered consciousness.

Although the severity of a movement disorder can differ from one person to another and, in the case of seizures, from episode to episode, it is widely acknowledged that experiencing a short seizure or a life-long movement disorder is debilitating. For example, a seizure may be experienced as a trance-like state from a few seconds up to several minutes or as lost consciousness and convulsions whereas a movement disorder may be experienced as involuntary and prolonged muscle contraction. Regardless of the severity of the seizure or movement disorder, their occurrence has significant consequences; these can vary from limited ability to participate in certain activities or perform certain tasks, such as driving, to severe impairment and effective incapacitation.

Moreover, there are usually external risks associated with experiencing a seizure or a movement disorder in general; for example a person experiencing a seizure or movement disorder may choke, break a bone, etc; thus, timely detection is paramount. Further, prevention and characterisation of individual-specific seizures as well as reduction of symptoms of chronic movement disorders would be highly advantageous to medical practitioners, carers and families of people who suffer from seizures or movement disorders.

WO2007/034476 discloses a device and a method for detecting an epileptic event by attaching a watch-like device on a user's limb, which watch-like device comprises a detector including three accelerometers which determine whether a seizure is occurring by comparing motion signal parameters to non-epileptic and epileptic movement parameters. While this system detects a paroxysmal event once it has started, the system does not predict or prevent it. As a result, the system is capable of evaluating whether the subject requires an alert, assistance or medication only after the event has started. In addition, the system is limited to certain types of seizures due to its dependency on limited types and point of signal collections.

Further, prior art systems experience a high rate of false positives when detecting an event. For example, the device and method disclosed in WO2007/034476 only calculates the probability of a seizure occurring at a specific time by using three-dimensional motion detectors; thus, sudden movements may generate a false positive alert. In addition, the device and method disclosed therein only detect changes to movement patterns.

WO2009/020880 discloses apparatus for diagnosing epileptic and non-epileptic movements of a subject during seizures by using a movement detection device connected to a processor module. Software or firmware algorithms operate within the processor module for describing and characterising the statistical properties of the movement signal during clinical seizure episodes. The system can be used to distinguish the movement features of epileptic seizure events from those of non-epileptic pseudo-seizures. The movement detection device may be an accelerometer, an electromyographic electrode or an optoelectronic distortion sensor. In an embodiment, the subject may be placed in a clinical setup in which doctors monitor the apparatus and compare the results obtained from the apparatus with those obtained from an electroencephalogram used in addition to or instead of the apparatus.

EP2123221 describes a seizure detection system comprising a motion detector, cameras, a communications line and a processor and/or seizure alert system; the motion detector being an accelerometer or gyro sensor. The system allows differentiation of seizure associated motion from non-seizure associated motion.

WO2007/034476 relates to a device and method for detecting and alerting of an epileptic seizure. The device comprises at least one motion sensor adapted to produce an electrical signal corresponding to a mechanical movement of the device, a signal processing unit adapted to compare at least one parameter from a signal output with at least one set of stored sets of motion signal parameters, a communication unit adapted to transmit an alert signal to a remote location and a control circuit adapted to activate the communication unit when the signal output meets a threshold level.

WO2002/082999 discloses a method and device for detecting and interpreting the motion of a user from a sequence of images. The device comprises an optical system adapted to capture an image, an image collector adapted to convert the image into an image signal, and a control unit adapted to record a plurality of image signals. An image database adapted to store raw data and an image processor adapted to analyse image data may be connected to the device. The device has a discrimination function for detecting and characterising periodic movement of a bed or wheelchair ridden subject to improve identification of epileptic seizures and non-epileptic events.

Moreover, current systems and studies require prolonged monitoring, typically of image data, by a medical practitioner. In addition, the data, generally video images, must be viewed in person by medically trained staff and processed to obtain user-specific data which is then compared to standard parameters. This is both time consuming and very expensive.

The present system seeks to provide a system adapted to monitor symptoms associated with movement disorders or seizures, which can then be used to predict, detect, analyse, characterise and or prevent or subdue said symptoms.

According to the present invention there is provided a system with the characteristics of claim 1. Preferred aspects of the invention can be found in the subclaims.

In contrast to prior art methods and devices, the present system is capable of accurately monitoring thereby allowing to detect, predict, characterise and classify seizures or movement disorders symptoms. Further, the present system is suitable for analysing, preventing or subduing seizures or movement disorder symptoms and not only of monitoring or detecting them.

Moreover, the present invention detects whether a subject is experiencing other symptoms or environmental factors which are related to or trigger a seizure or movement disorder symptoms by collecting movement data to monitor and prevent movement disorder symptoms or seizures; i.e., the system functions as a unified integrated platform for applications requiring collection of movement and environmental data. In a preferred embodiment, the system uses additional sensors, such as humidity sensors, in addition to known physical sensors adapted to measure vital signs. Further, the system may include an input from a on-invasive blood-glucose level measurement apparatus, such as, for example, the apparatus described in WO 2002/082983. In addition, facial reaction and eye movement patterns may be used to detect changes associated with a seizure or symptoms of a movement disorder. As a result, the present system allows medical practitioners, users and their families to analyse and classify seizures or movement disorders. The system may also incorporate movement trajectory and posture detection algorithms based on sonar based localisation techniques amplified with acceleration sensors, such as, for example, AliveTech (RTM) sensors, to enhance and broaden the range of movement disorders which can be detected.

In contrast to available systems, the present invention can acquire data in three different modes: online, offline and hybrid mode. While in online acquisition mode, the system acquires real-time data relating to the user's body and its surrounding environment which is subsequently recorded locally or relayed in real-time; this enables the system to predict, detect and monitor movement disorder symptoms or seizure. On the other hand, when the system is in offline mode, the data is directly or remotely collected from pre-recorded media which is then relayed to the appropriate module or unit; this mode is useful in characterising seizures or movement disorder symptoms of a specific user, i.e. it allows individualisation of standard data. This mode also enables analysis of the acquired data which can subsequently be used for predicting, detecting, preventing or subduing a seizure or movement disorder symptoms. Further, the data can be obtained while the user carries out his normal activities and recorded; as a result, there is no time wastage nor is a medical practitioner's presence required, therefore, the cost of and inconvenience caused by the process are significantly reduced. Finally, when the system is in hybrid acquisition mode the data is retrieved directly from sensors as well as indirectly from pre-recorded media, i.e. the two types of data acquisition are combined. This mode is particularly useful for predicting, preventing and subduing seizures or symptoms because actual data can be compared with user-specific recorded data; thus, the system is capable of predicting likelihood of a seizure or movement disorder symptoms under in a particular environment; in other words, the system uses individualised standard data and user-specific data to predict a seizure or symptoms of a movement disorder in real time and may be used to prevent or subdue them if the user has been prescribed medication which is delivered through the system.

It has been observed that seizures or movement disorder symptoms are sometimes triggered or exacerbated by a change in light or temperature, sudden activity, excitement or stress. Thus, if the user of this system is affected by changes to light or temperature, sudden activity, excitement or stress, the system will automatically recognise that the user is susceptible to experience a seizure or symptoms if differences in the environment or the user's vital signs are detected. Further, the system may alert the user or a remote monitoring unit that conditions have changed and that he is more susceptible to experience a seizure or movement disorder symptoms. In addition, the system may deliver prescribed medication to the user automatically under these circumstances.

For example, the system may be used to predict asthma-related seizures; if the user suffers from severe attacks, these may be triggered by changes in atmospheric humidity or particular chemicals, such as tobacco and tar smoke (i.e. cigarette smoke). It is also widely accepted that stress may also trigger an attack. As the present system monitors humidity, odours and heart rate, it is capable of sending an alert signal to the user or to a remote location when the user is susceptible to experience a severe attack which may eventually lead to a seizure. Moreover, the system may deliver prescribed medication to the user automatically. In the same way, if a user is epileptic and seizures are triggered by stress or a sudden change in light, the system monitors the environment and sends an alert signal to the user or a remote location in the event the user is deemed likely to experience a seizure. Thus, the system is capable of using real time data to predict the likelihood of a seizure or movement disorder symptoms occurring under specific circumstances for a specific user.

An exemplary embodiment of the present invention will now be described in detail with reference to the following figures.
Figure 1 is a block diagram of the system.
Figure 2 is a flow diagram of the path followed by the data once it is detected and analysed.

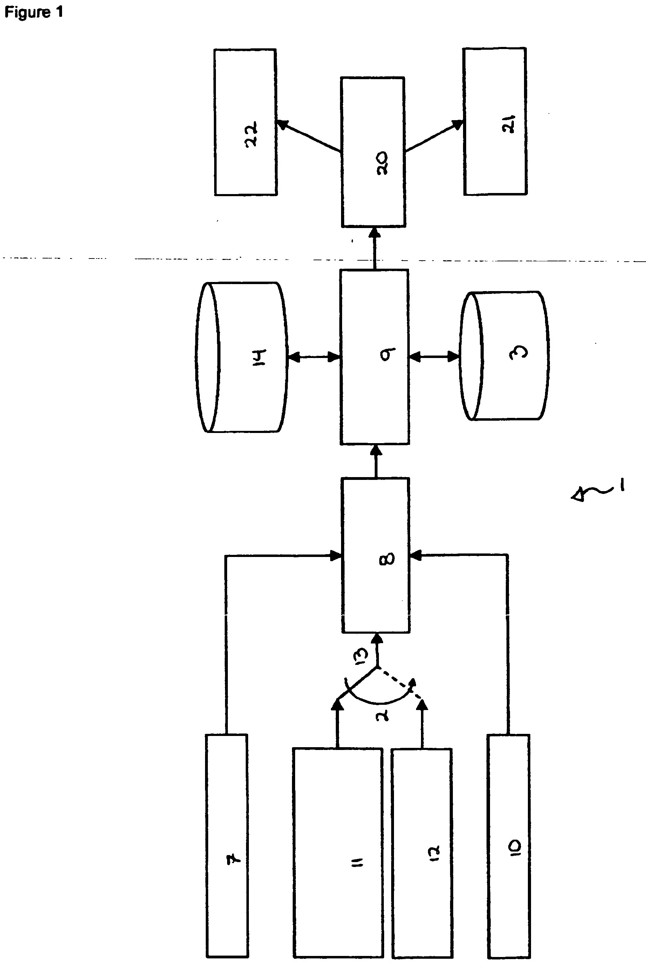

Referring to Figure 1, the monitoring system 1 comprises data acquisition means 2 having at least one movement detector 6, typically accelerometers or gyroscopic methods, recording means 3, typically video recording means 3 and a signal processing unit, typically a CPU or a wireless network, having a central aggregation and relaying module 8 and a processing module 9. The system 1 further comprises other sensors or data acquisition means 7 for environment and additional user data acquisition; for example, a light sensor, a temperature sensor, a humidity sensor, a smell detector, heart rate monitoring means, blood pressure monitoring means, blood-glucose level monitoring means (non-invasive), electrocardiography means, electroencephalography means and acoustic distance measurement devices. The type of sensor will depend at least to some extent on the indication being monitored. The system further comprises location-sensing means 10, such as GPS, WiFi or equivalent positioning means.

During online acquisition mode 11 a plurality of sensors 7 are connected to the subject to acquire data relating to movement and the subject's body (for example, pulse, temperature, respiration rate, blood pressure, blood glucose levels, electrical activity of the brain, as measured by an electroencephalogram (EEG), or the heart, as measured by an electrocardiogram (ECG). The sensor output is then recorded locally by the recording means 3 or relayed in real-time to a central data aggregation and relaying module 8 for signal processing.

When the system 1 acquires data in the offline mode 12, the data is directly or remotely collected from pre-recorded media, such as video recordings of the subject, recorded test data, sound recordings of the patient, environmental data recordings, etc. The sensor output is subsequently relayed to the central aggregation and relaying module 8 for signal processing.

If the system is in hybrid acquisition mode 13, data will be retrieved directly from sensors 6, 7 attached to the user as well as indirectly from pre-recoded media. The sensor output and the output of the pre-recorded media are sent to the central aggregation and relaying module 8 which module combines the two types of output to enable the processing module 9 to characterise the data. The data collected is useful in the analysis and classification of movement data; in addition, it plays a substantial role in generating and transmitting an alert signal.

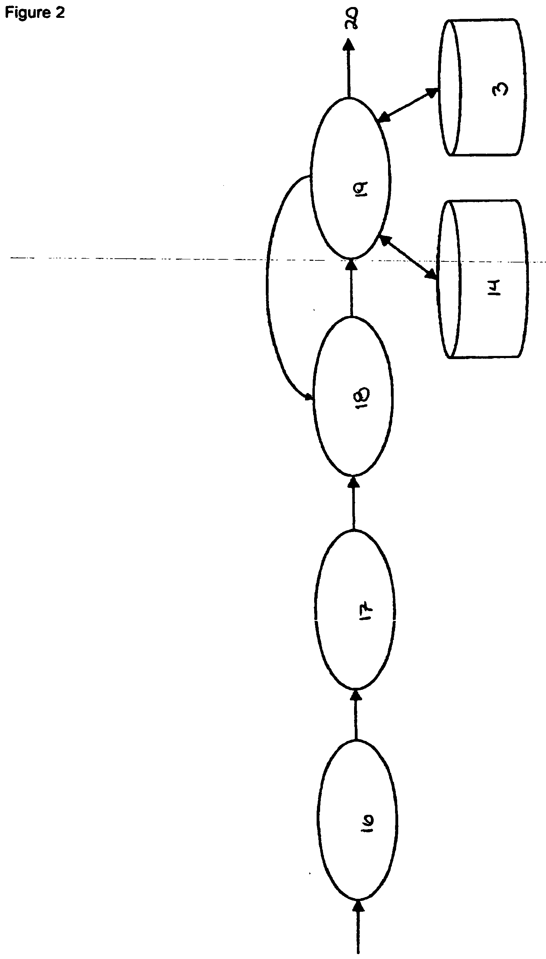

The output of the data acquisition means 2 in any of its three functioning modes is then transferred to the central data aggregation and relaying module 8 which combines and pre-processes the collected output to reduce data size and increase its accuracy. This module 8 cross-references sensor or pre-recorded media outputs with a database 14 including standard seizure or movement disorder data and patient-specific data so that data relating to regular movements not associated with seizures or duplicated data is disregarded and it compresses data to ensure further analysis thereof is performed more efficiently. Subsequently, this module relays an aggregated output to the processing module 9 connected to said aggregation and relaying module via an internal connection or a wireless network, which wireless network enables a remote connection over large coverage areas; for example a mobile telephone system, WiFi, WiMax. If a wireless network is used, location information data is collected using GPS systems and/or through other localisation means 10 depending on the technology used for wireless connection between the aggregation and relaying module 8 and the processing module 9. Either method can be used separately or in combination to allow better coverage (indoor and outdoor) and enhanced accuracy. The location sensing means 10 can be incorporated in the data aggregation and relaying module 8. The processing module then processes the signal as described in Figure 2 to generate a result output signal 20 comprising information on whether the movement is symptomatic, asymptomatic or predictive of imminent symptoms; subject-specific information, a proposed course of action and a information on whether an alert is necessary.

In the event that the movement is symptomatic or suggests imminent symptoms, the processing module 9 sends an alert signal to a generation and propagation module 21. The alert generation and propagation module 21 can be remotely configured by monitoring staff or the user to send an alert or a plurality of alerts, including sound, visual and data alerts or a combination thereof, to designated monitoring means or a predetermined person to inform said monitoring means or predetermined person that the user is either experiencing a seizure or movement disorder symptoms or is under specified conditions defined by the system which make symptoms imminent. As the alert signal contains information on the type of movement, subject-specific information and a suggested course of action the system either sends an alert to the subject so he can follow the suggested action or, if the system is used with an automated drug delivery control system 22, the alert triggers the automated drug delivery control system 22 so a drug is delivered or treatment dosages readjusted based on the monitored conditions. Further, said alert signal may provide the location of the user.

Figure 2 shows the steps followed to process the aggregated output signal. Firstly, the processing module 9 filters 16 signals to remove background noise and signal artefacts as the data collected from the plurality of sensors 6, 7 or pre-recorded media 3 will include noise signals. During filtering 16, the processing module 9 removes background noise signals, both user-specific and environmental, from input signals collected from different sensors 6, 7 by removing signals corresponding to predetermined frequencies and speeds. Further, noise resulting from imperfections in the sensing process are also removed at this stage. It has been observed that epileptic fit spans have a characteristic frequency and speed of motion. It is also known that voluntary repetitive movement, walking for example, differs in both frequency and speed of motion when compared to seizure movement; thus, removing signals unrelated to the seizure or movement disorder symptoms allows the system to concentrate exclusively on critical data. Thus, the present system filters time domain, speed domain and frequency domain to remove unwanted signals by applying knowledge on expected data through the use of a combination of threshold based and correlation based methods to filter out undesired signals, i.e. it compares the time, speed and frequency of movement in the aggregated signals against a database including voluntary movement data. This process enhances the accuracy of the eventual actions initiated by the system. The aggregated signal output is correlated with standard data and patient-specific data stored in a database to establish a movement threshold; alternatively, a predetermined threshold may be used. If any signal included in the output is under the applied threshold it is filtered out whereas if the signal exceeds the applied threshold it continues to the following processing step.

Secondly, the processing module processes and analyses signals to expose important features or properties of the aggregated data 17. During this step, various signal transformation methods, in particular, fractional Fourier transform, wavelet transform or short-time Fourier transform are applied to the signals to expose dominant frequencies, statistical correlations and time-frequency correlations, in other words, the transform methods allow the system to determine whether the signal correlates to the characteristics of a seizure or movement disorder. Once the transform methods have been applied, additional filtering will remove noise signals which could not previously be separated from data signals; only the transformed data signals will proceed to the subsequent step.

Thirdly, the processing units recognises symptomatic movement patterns in the signals 18 by applying dynamically adaptive customized fuzzy logic algorithms with tuneable parameters to the filtered and processed signals; which parameters are set by using standard movement data, user specific data, and environmental data to perform movement pattern recognition 18 and classification of movement signals into predefined categories obtained by analysing standard seizure or disorder data and user specific data. The processing module 9 provides the system with parameters for the fuzzy logic algorithms and the predefined categories dynamically, i.e. in real time.

Fourthly, the processing module allows the signals to be analysed and classified 19 by using machine-learning algorithms which use training, learning and medical knowledge based data 14 to allow fine-tuning of the classification process 19. Once the signals have been subjected to the pattern recognition 18 step, the data processing module 9 applies machine-learning algorithms to the analysed and classified signals (i.e. the signals received from the pattern recognition step) with the purpose of deciding whether the data is associated with a medical condition 19. These machine-learning algorithms utilise medical databases 14, historical data in the recording means 3, and the classified signal data generated by the pattern recognition step 18 to generate a result which indicates whether the signals from the monitored movement data are associated with a medical condition. The processing module 9 detects or predicts movements associated with seizures or movement disorders by using a wavelet basis extracted from a learned movement model, which analyses time, speed and frequency signals to determine whether the movement under analysis is associated with seizures, or movement disorders. The algorithm then analyses the difference between predetermined upper and lower energy correlation thresholds to detect symptomatic movements. If the signals from the observed movement result in a difference between the predetermined thresholds which exceeds predefined values, the observed movement is classified as symptomatic whereas if the difference does not exceed predetermined values but is within another predefined range, the observed movement is classified as voluntary movement. The system is further adapted to identify differences which correlate to a developing seizure or symptomatic episode. Once the movement observed is classified as symptomatic, asymptomatic or predictive of imminent symptoms, the processing module 9 will correlate the movement signal to the medical database medical data 14, historical database 3, and classified signal data generated by the pattern recognition step 18 and will generate a result output signal 20 comprising information on whether the movement is symptomatic, asymptomatic or predictive of imminent symptoms; subject-specific information, a proposed course of action and a information on whether an alert is necessary.

In the event that the movement is symptomatic or suggests imminent symptoms, the processing module 9 will send an alert signal to a generation and propagation module. The alert generation and propagation module can be remotely configured by monitoring staff or the user to send an alert or a plurality of alerts, including sound, visual and data alerts or a combination thereof, to designated monitoring means or a predetermined person to inform said monitoring means or predetermined person that the user is either experiencing a seizure or movement disorder symptoms or is under specified conditions defined by the system which make symptoms imminent. As the alert signal contains information on the type of movement, subject-specific information and a suggested course of action the system either sends an alert to the subject so he can follow the suggested action or, if the system is used with an automated drug delivery control system, the alert triggers the automated drug delivery control system so a drug is delivered or treatment dosages readjusted based on the monitored conditions. Further, said alert signal may provide the location of the user.

The output signal is also used by the system as tuning feedback output; thus, it is sent back to the processing module 9 to maintain or improve fuzzy logic parameters and enhance classification accuracy during the pattern recognition step 18.

During trials, the system of the present invention successfully detected 92 episodes out of 100. Taking into account variations of movement and differences between patients, the overall rate of detection was found to be approximately 90%. The same trials, established that the system experiences a false alarm probability of around 15% when considering fast, repetitive movements caused by activities, such as, throwing a ball, playing tennis, running, jogging, or similar actions. Thus, the present system provides a clear advantage over prior art devices.

## Claims

1. A monitoring system for monitoring movements of a user comprising data acquisition means (2), recording means (3), and a signal processing unit;
wherein said data acquisition means (2) comprises at least one movement detector (6) for detecting movement data of a user and generating a movement signal, the characteristics of which movement signal are dependent on the characteristics of the movement of the user;
wherein said signal processing unit is configured to receive said movement signal, to filter noise signals and voluntary movement signals from said
movement signal to generate a filtered signal, and to analyse said filtered signal to recognise symptomatic movement patterns;
the data acquisition means (2) further comprising additional sensors (7) for detecting additional data capable of reflecting other user's symptoms or environmental factors which are related to or are capable of triggering a seizure
or movement disorder symptoms, and generating respective additional signals is the signal processing unit being further configured to use said additional data to generate a result output signal (20) predicive of the likelihood of a seizure or movement disorder symptoms occurring.

2. A system according to claim 1, wherein the processing unit (2) further comprises a central aggregation and relaying module (8) and a processing module (9); said central aggregation and relaying module (8) being configured to combine and pre-process the signals generated by the data acquisition means (2) and to relay an aggregated signal (16) to the processing module (9), and said processing module being configured to process the aggregated signal (16) to generate the result output signal (20).

3. A system according to claim 2, wherein the data acquisition means (2) is configured to operate in an online mode (11), in which online mode (11) said data acquisition means (2) are connected to a user to acquire data to produce online signals, which online signals are recorded locally by recording means (3) or relayed in real time to the central data aggregation and relaying module (8) for signal processing.

4. A system according to claim 2, wherein said data acquisition means (2) is configured to operate in an offline mode (12), in which offline mode (12) data is directly or remotely collected from recording means (3) to produce offline signals, which offline signal are relayed to the central aggregation and relaying module (8) for signal processing.

5. A system according to claim 2, wherein data acquisition means (2) is configured to operate in an hybrid mode (13), in which hybrid mode (13) data is retrieved directly from said data acquisition means (2) attached to a user and indirectly from recording means (3) to produce hybrid signals comprising online and offline signals which hybrid signals are sent to the central aggregation and relaying module (8) for signal processing.

6. A system according to any one of claims 2 to 5, when dependent on claim 2, wherein the data acquisition means (2) or the data aggregation and relaying module (8) comprise locations sensing means (10) such as GPS, WiFi or equivalent positioning means.

7. A system according to any of the preceding claims, wherein the additional sensors (7) are selected from: a light sensor, a temperature sensor, a humidity sensor, a smell detector, heart rate monitoring means, blood pressure monitoring means, non-invasive blood-glucose level monitoring means, electrocardiography means, electroencephalography means or an acoustic distance measurement device.

8. A system according to any one of claims 2 to 7, when dependent on claim 2, wherein the central data aggregation and relaying module (8) is further configured to compress the signals generated by the data acquision means (2) and to increase accuracy of the output by filtering out data relating to normal voluntary movements and duplicated data.

9. A system according to claim 8, wherein the signal is time domain and frequency domain filtered to recognise patterns therein associated with a predetermined condition.

10. A system according to claims 8 or 9, wherein the processing module (9) is configured to recognise patterns in the aggregated signal by using fuzzy logic methods or machine learning algorithms, which algorithms analyse the difference between a predetermined upper and lower energy correlation thresholds to thereby characterise and classify said aggregated signal, detect or predict predetermined movements and to generate a result output signal (20) which indicates whether the signals generated by the data acquisition means (2) are associated with a predetermined condition.

11. A system according to any one of claims 2 to 10, when dependent on claim 2, wherein the processing module (9) is further configured to generate an alert signal, which alert signal is transferred to an alert generation and propagation module (21), which alert generation and propagation module (21) is configured to send an alert or a plurality of alerts to designated remote monitoring means or a predetermined person when the signals generated by the data acquisition means (2) are associated with a predetermined condition.

12. A system according to claim 11, wherein the alert signal provides the location of the user.

13. A system according to any of the preceding claims, further comprising an automated drug delivery system (22), wherein in the event that a seizure or movement disorder symptoms are detected or predicted, an instruction is sent to the automated drug delivery system (22) to allow delivery of an appropriate dose.

## Patentansprüche

1. Überwachungssystem zur Überwachung von Bewegungen eines Benutzers, umfassend ein Datenerfassungsmittel (2), ein Aufzeichnungsmittel (3) und eine Signalverarbeitungseinheit;
wobei das besagte Datenerfassungsmittel (2) mindestens einen Bewegungsmelder (6) zur Erfassung von Bewegungsdaten eines Benutzers und zur Erzeugung eines Bewegungssignals umfasst, dessen Eigenschaften von den Eigenschaften der Bewegung des Benutzers abhängen;
wobei die besagte Signalverarbeitungseinheit zum Empfang des besagten Bewegungssignals, zum Ausfiltern von Rauschsignalen und willkürlichen Bewegungssignalen aus dem besagten Bewegungssignal zwecks Erzeugung eines gefilterten Signals und zur Analyse des besagten gefilterten Signals zwecks Erkennung von symptomatischen Bewegungsmustern ausgelegt ist; wobei das Datenerfassungsmittel (2) weiter zusätzliche Sensoren zur Erfassung von zusätzlichen Daten, die andere Benutzersymptome oder Umweltfaktoren, die mit der Auslösung eines Anfalls oder von Bewegungsstörungssymptomen verbunden sind oder diese auslösen können, widerspiegeln, und zur Erzeugung von entsprechenden zusätzlichen Signalen umfasst;
wobei die Signalverarbeitungseinheit weiter zur Benutzung der besagten zusätzlichen Daten zur Erzeugung eines Ergebnis-Ausgangssignals (20) zur Voraussage der Wahrscheinlichkeit eines Anfalls oder von Bewegungsstörungssymptomen ausgelegt ist.

2. System nach Anspruch 1, wobei die Verarbeitungseinheit (2) weiter ein zentrales Sammel- und Weiterleitmodul (8) und ein Verarbeitungsmodul (9) umfasst; wobei das besagte zentrale Sammel- und Weiterleitmodul (8) zur Kombination und Vorverarbeitung der vom Datenerfassungsmittel (2) erzeugten Signale und zur Weiterleitung eines Sammelsignals (16) an das Verarbeitungsmodul (9) ausgelegt ist und das besagte Verarbeitungsmodul zur Verarbeitung des Sammelsignals (16) zwecks Erzeugung des Ergebnis-Ausgangssignals (20) ausgelegt ist.

3. System nach Anspruch 2, wobei das Datenerfassungsmittel (2) zur Arbeit in einem Online-Modus (11) ausgelegt ist, in dem das besagte Datenerfassungsmittel (2) zur Erfassung von Daten und zur Erzeugung eines Online-Signals an einen Benutzer angeschlossen ist und die Online-Signale örtlich vom Aufzeichnungsmittel (3) aufgezeichnet oder in Echtzeit zur Signalverarbeitung an das zentrale Datensammel- und Weiterleitmodul (8) weitergeleitet werden.

4. System nach Anspruch 2, wobei das besagte Datenerfassungsmittel (2) zur Arbeit in einem Offline-Modus (12) ausgelegt ist, in dem Daten zur Erzeugung von Offline-Signalen, die zur Signalverarbeitung an das zentrale Sammel- und Weiterleitmodul (8) weitergeleitet werden, direkt oder aus der Ferne aus dem Aufzeichnungsmittel (3) gesammelt werden.

5. System nach Anspruch 2, wobei das Datenerfassungsmittel (2) zur Arbeit in einem Hybrid-Modus (13) ausgelegt ist, in dem Daten zur Erzeugung von aus Online- und Offline-Signalen bestehenden Hybrid-Signalen, die zur Signalverarbeitung an das zentrale Sammel- und Weiterleitmodul (8) weitergeleitet werden, direkt aus dem an einen Benutzer angeschlossenen besagten Datenerfassungsmittel (2) oder indirekt aus dem Aufzeichnungsmittel (3) gewonnen werden.

6. System nach einem der Ansprüche 2 bis 5, falls abhängig von Anspruch 2, wobei das Datenerfassungsmittel (2) oder das Weiterleitmodul (8) Positionsgebermittel (10) wie z.B. GPS, WiFi oder gleichwertige Positioniermittel umfasst.

7. System nach einem der vorhergehenden Ansprüche, wobei die zusätzlichen Sensoren (7) aus folgenden Möglichkeiten ausgewählt werden: einem Lichtsensor, einem Temperatursensor einem Feuchtigkeitssensor, einem Geruchsmelder, einem Pulsmessgerät, einem Blutdrucküberwachungsmittel, einem nichtinvasiven Blutzuckerwertüberwachungsmittel, einem Elektrokardiografiemittel, einem Elektroenzephalografiemittel oder einem akustischen Entfernungsmessgerät.

8. System nach einem der Ansprüche 2 bis 7, falls abhängig von Anspruch 2, wobei das zentrale Datensammel- und Weiterleitmodul (8) weiter zur Kompression der vom Datenerfassungsmittel (2) erzeugten Signale und zur Steigerung der Ausgangsgenauigkeit durch Ausfiltern von mit normalen willkürlichen Bewegungen verbundenen Daten und von duplizierten Daten ausgelegt ist.

9. System nach Anspruch 8, wobei das Signal zur Erkennung von darin enthaltenen mit einem vorgegebenen Zustand verbundenen Mustern zeitdomänen- und frequenzdomänengefiltert wird.

10. System nach Anspruch 8 oder 9, wobei das Verarbeitungsmodul (9) zur Erkennung von Mustern im Sammelsignal durch Anwendung von Fuzzylogik-Verfahren oder Maschinenlernalgorithmen ausgelegt ist, die den Unterschied zwischen einer vorgegebenen oberen und unteren Energiekorrelationsschwelle analysieren und dadurch das besagte Sammelsignal bezeichnen und einstufen, vorgegebene Bewegungen erfassen oder voraussagen und ein Ergebnis-Ausgangssignal (20) erzeugen, das angibt, ob die vom Datenerfassungsmittel (2) erzeugten Signale mit einem vorgegebenen Zustand verbunden sind.

11. Verfahren nach einem der Ansprüche 2 bis 10, falls abhängig von Anspruch 2, wobei das Verarbeitungsmodul (9) weiter zur Erzeugung eines Warnsignals ausgelegt ist, das zu einem Warnungserzeugungs- und -übertragungsmodul (21) weitergeleitet wird, das zum Senden einer Warnung oder einer Vielzahl von Warnungen an bestimmte Fernüberwachungsmittel oder eine vorgegebene Person ausgelegt ist, wenn die vom Datenerfassungsmittel (2) erzeugten Signale mit einem vorgegebenen Zustand verbunden sind.

12. Dystam nach Anspruch 11, wobei das Warnsignal die Position des Benutzers bereitstellt.

13. System nach einem der vorhergehenden Ansprüche, weiter umfassend ein automatisiertes Medikamentenverabreichungssystem (22), wobei bei Erkennung oder Voraussage eines Anfalls oder von Bewegungsstörungssymptomen an das Medikamentenverabreichungssystem (22) eine Anweisung zur Freigabe einer angemessenen Dosis gesendet wird.

## Revendications

1. Un système de surveillance destiné à suivre les mouvements d'un utilisateur et comprenant des moyens d'acquisition de données (2), des moyens d'enregistrement (3) et une unité de traitement de signaux ;
dans lequel lesdits moyens d'acquisition de données (2) comportent au moins un détecteur de mouvement (6) afin de détecter les données de mouvement d'un utilisateur et de générer un signal de mouvement, les caractéristiques de ce signal de mouvement dépendant des caractéristiques de mouvement de l'utilisateur ;
dans lequel ladite unité de traitement de signaux est configurée pour recevoir ledit signal de mouvement, pour filtrer les signaux de bruit et les signaux de mouvement volontaire provenant de ce signal de mouvement afin de générer un signal filtré, et pour analyser ce signal filtré afin de reconnaître les schémas de mouvements symptomatiques ;
les moyens d'acquisition de données (2) comprenant également d'autres capteurs (7) destinés à détecter des données supplémentaires susceptibles de refléter d'autres symptômes de l'utilisateur ou des facteurs environnementaux liés à, ou susceptibles de déclencher une crise d'épilepsie ou des symptômes de troubles du mouvement, et à générer les signaux supplémentaires correspondants ;
l'unité de traitement de signaux étant en outre configurée pour utiliser lesdites données supplémentaires afin de générer un signal sortant de résultat (20) prévoyant la probabilité de survenue d'une crise d'épilepsie ou de symptômes de troubles du mouvement.

2. Un système selon la revendication 1, dans lequel l'unité de traitement (2) comprend en outre un module central d'agrégation et de relais (8) et un module de traitement (9) ; ledit module central d'agrégation et de relais (8) étant configuré pour combiner et prétraiter les signaux générés par les moyens d'acquisition de données (2) et pour transmettre un signal agrégé (16) au module de traitement (9), et ledit module de traitement étant configuré pour traiter le signal agrégé (16) afin de générer le signal sortant de résultat (20).

3. Un système selon la revendication 2, dans lequel les moyens d'acquisition de données (2) sont configurés pour fonctionner en mode en ligne (11) ; dans ce mode en ligne (11), les moyens d'acquisition de données (2) sont connectés à un utilisateur afin d'acquérir des données et produire des signaux en ligne, lesquels sont enregistrés localement par des moyens d'enregistrement (3) ou transmis en temps réel au module central d'agrégation et de relais de données (8) en vue du traitement des signaux.

4. Un système selon la revendication 2, dans lequel lesdits moyens d'acquisition de données (2) sont configurés pour fonctionner en mode hors ligne (12) ; dans ce mode hors ligne (12), les données sont collectées directement à partir des moyens d'enregistrement (3) ou à distance de ceux-ci afin de produire des signaux hors ligne, lesquels sont transmis au module central d'agrégation et de relais (8) en vue de leur traitement.

5. Un système selon la revendication 2, dans lequel les moyens d'acquisition de données (2) sont configurés pour fonctionner en mode hybride (13) ; dans ce mode hybride (13), les données sont récupérées directement à partir desdits moyens d'acquisition de données (2) fixés sur un utilisateur et indirectement à partir des moyens d'enregistrement (3) afin de produire des signaux hybrides composés de signaux en ligne et hors ligne, lesquels sont envoyés vers le module central d'agrégation et de relais (8) en vue de leur traitement.

6. Un système selon l'une quelconque des revendications 2 à 5, lorsqu'elles dépendent de la revendication 2, dans lequel les moyens d'acquisition de données (2) ou le module d'agrégation et de relais de données (8) comprennent des moyens de localisation (10) de type GPS, WiFi ou équivalents.

7. Un système selon l'une quelconque des revendications précédentes, dans lequel les capteurs supplémentaires (7) sont sélectionnés parmi les suivants : un capteur de lumière, un capteur de température, un capteur d'humidité, un capteur d'odeurs, des moyens de suivi du rythme cardiaque, des moyens de suivi de la tension artérielle, des moyens de suivi du taux de glycémie non invasif, des moyens d'électrocardiographie, des moyens d'électroencéphalographie ou un dispositif de mesure de la distance acoustique.

8. Un système selon l'une quelconque des revendications 2 à 7, lorsqu'elles dépendent de la revendication 2, dans lequel le module central d'agrégation et de relais de données (8) est en outre configuré pour compresser les signaux générés par les moyens d'acquisition de données (2) et pour améliorer la précision du résultat en excluant les données relatives aux mouvements volontaires normaux et les données en double.

9. Un système selon la réclamation 8, dans lequel le signal est filtré par domaine temporel et fréquentiel afin d'y reconnaître les schémas associés à un état prédéterminé.

10. Un système selon les revendications 8 ou 9, dans lequel le module de traitement (9) est configuré pour reconnaître des schémas dans le signal agrégé en utilisant des méthodes de logique floue ou des algorithmes d'apprentissage automatique ; ces algorithmes analysent la différence entre des seuils de corrélation énergétique inférieur et supérieur prédéterminés afin de caractériser et classifier ledit signal agrégé, détecter ou prédire des mouvements prédéterminés et générer un signal sortant de résultat (20) qui indique si les signaux générés par les moyens d'acquisition de données (2) sont associés à un état prédéterminé.

11. Un système selon l'une quelconque des revendications 2 à 10, lorsqu'elles dépendent de la revendication 2, dans lequel le module de traitement (9) est en outre configuré pour générer un signal d'alerte, lequel est transmis à un module de génération et de propagation d'alerte (21), qui est configuré pour envoyer une ou plusieurs alertes vers des moyens de suivi à distance désignés ou une personne prédéterminée lorsque les signaux générés par les moyens d'acquisition de données (2) sont associés à un état prédéterminé.

12. Un système selon la revendication 11, dans lequel le signal d'alerte indique la localisation de l'utilisateur.

13. Un système selon l'une quelconque des revendications précédentes, qui comprend en outre un système d'administration de médicaments automatique (22) qui, en cas de détection ou de prédiction d'une crise d'épilepsie ou de symptômes de troubles du mouvement, envoie des instructions au système d'administration de médicaments automatique (22) pour permettre l'administration d'une dose appropriée.
